# EUROPEAN PATENT APPLICATION

(11) **EP 4 109 465 A1**
(43) Date of publication of application: **28.12.2022**
(21) Application number: 20919891.0
(22) Date of filing: 04.12.2020
(51) Int. Cl.: G16H 40/60

(54) **METHOD AND SYSTEM FOR MANAGING THERAPY PLAN DATA, AND DATA EXCHANGE DEVICE**

(30) Priority: 17.02.2020 CN 202010097538
(71) Applicant: Neuboron Therapy System Ltd., Xiamen, Fujian 361026 (CN)
(72) Inventor: ZHANG, Xiao, Fujian Province, 361026 P.R. (CN)
(74) Representative: Gunzelmann, Rainer
(86) International application number: PCT/CN2020/133830
(87) International publication number: WO 2021/164372

(57) **Abstract**

A method and system for managing therapy plan data, and a data exchange device. The method for managing therapy plan data comprises: receiving first therapy plan data transmitted by a therapy plan device by means of a first communication protocol (S 110); and transmitting the first therapy plan data to a therapy control device by means of a second communication protocol (S120), thereby allowing for timely data interaction between the therapy plan device and the therapy control device.

## Description

### TECHNICAL FIELD

The disclosure relates to the technical field of therapy plan data management, and in particular to a method and system for managing therapy plan data and a data exchange device.

### BACKGROUND

Existing therapy plan device and therapy control device are two independent devices, which are operated by different users and are set with different authorities. When data exchange is performed directly between the therapy plan device and the therapy control device, as both the therapy plan device and the therapy control device are systems operated by the user directly, they may be affected by external factors, for example, the therapy plan device and the therapy control device may not be online simultaneously due to malicious data tampering, electricity outage, network outage and the like, so that the therapy control device is unable to acquire data from the therapy plan device timely. In addition, as both the therapy plan device and the therapy control device are mature systems, the two mature systems are poor in compatibility there-between, and problems such as low efficiency, poor maintainability, low system redundancy and the like may occurs when data interaction is performed directly between the two systems.

### SUMMARY

In order to solve the above-mentioned technical problem, embodiments of the disclosure provide a method and system for managing therapy plan data and a data exchange device, thereby achieving data interaction between the therapy plan device and the therapy control device timely.

In a first aspect of the embodiments of the disclosure, the embodiments of the disclosure provide a method for managing therapy plan data, including the following operations. First therapy plan data transmitted by a therapy plan device by means of a first communication protocol is received; and the first therapy plan data is transmitted to a therapy control device by means of a second communication protocol.

In an embodiment of the disclosure, the method further includes the following operations. The first therapy plan data is stored; and the first therapy plan data is converted into second therapy plan data to allow the therapy control device to identify the second therapy plan data; herein the operation that the first therapy plan data is transmitted to the therapy control device by means of the second communication protocol includes: transmitting the second therapy plan data to the therapy control device by means of the second communication protocol.

In an embodiment of the disclosure, the method further includes the following operations. An order sent by the therapy control device is received; and the first therapy plan data is retrieved according to the order.

In an embodiment of the disclosure, the first communication protocol and the second communication protocol are the same or different.

In an embodiment of the disclosure, the first communication protocol or the second communication protocol is a communication protocol based on Internet Protocol (IP); further, the communication protocol based on IP includes a Transmission Control Protocol TCP/IP.

In an embodiment of the disclosure, the first communication protocol includes any one of a Hypertext Transfer Protocol (HTTP), a Hypertext Transfer Protocol Secure (HTTPS), a File Transfer Protocol (FTP) or a Secure Shell (SSH) Protocol, and the second communication protocol includes any one of the HTTP, the HTTPS, the FTP or the SSH protocol.

In a second aspect of the embodiments of the disclosure, the embodiments of the disclosure provide a data exchange device, including: a receiving module configured to receive first therapy plan data transmitted by a therapy plan device by means of a first communication protocol; and a transmission module configured to transmit the first therapy plan data to a therapy control device by means of a second communication protocol.

In an embodiment of the disclosure, the data exchange device further includes a database module configured to store the first therapy plan data, herein the receiving module is further configured to send the first therapy plan data to the database module, and the transmission module is further configured to retrieve the first therapy plan data from the database module.

In an embodiment of the disclosure, the data exchange device further includes a data conversion module configured to convert the first therapy plan data into second therapy plan data to allow the therapy control device to identify the second therapy plan data, herein the transmission module is further configured to transmit the second therapy plan data to the therapy control device by means of the second communication protocol.

In a third aspect of the embodiments of the disclosure, the embodiments of the disclosure provide a system for managing therapy plan data, including: a therapy plan device configured to generate first therapy plan data; a therapy control device configured to control therapy according to the first therapy plan data; and a data exchange device located between the therapy plan device and the therapy control device, and configured to receive the first therapy plan data transmitted by the therapy plan device by means of a first communication protocol and transmit the first therapy plan data to the therapy control device by means of a second communication protocol.

In an embodiment of the disclosure, the therapy plan device includes: a medical image acquisition module configured to acquire a medical image; a therapy plan forming module configured to form a therapy plan according to the medical image; a data generation module configured to generate the first therapy plan data according to the therapy plan; and a data output module configured to transmit the first therapy plan data to a receiving module in the data exchange device by means of the first communication protocol.

In an embodiment of the disclosure, the therapy control device includes: an order output module configured to send an order to a transmission module in the data exchange device by means of the second communication protocol; a data acquisition module configured to acquire, by means of the second communication protocol, the first therapy plan data transmitted by the transmission module in the data exchange device according to the order; and a therapy implementation module configured to control therapy according to the first therapy plan data.

In an embodiment of the disclosure, the data exchange device includes: a receiving module configured to receive the first therapy plan data transmitted by the therapy plan device by means of the first communication protocol; and a transmission module configured to transmit the first therapy plan data to the therapy control device by means of the second communication protocol. Preferably, the data exchange device further includes a database module configured to store the first therapy plan data, herein the receiving module is further configured to send the first therapy plan data to the database module, and the transmission module is further configured to retrieve the first therapy plan data from the database module. Further preferably, the data exchange device further includes a data conversion module configured to convert the first therapy plan data into second therapy plan data to allow the therapy control device to identify the second therapy plan data, herein the transmission module is further configured to transmit the second therapy plan data to the therapy control device by means of the second communication protocol.

According to the technical solutions provided by the embodiments of the disclosure, first therapy plan data transmitted by a therapy plan device by means of a first communication protocol is received; and the first therapy plan data is transmitted to a therapy control device by means of a second communication protocol, so that data interaction may be performed timely between the therapy plan device and the therapy control device, and problems such as low efficiency, poor maintainability, low system redundancy and the like occurred when data interaction is performed directly between the therapy plan device and the therapy control device are avoided.

### BRIEF DESCRIPTION OF THE DRAWINGS

FIG. 1 is a schematic flowchart of a method for managing therapy plan data provided by an embodiment of the disclosure.
FIG. 2 is a schematic flowchart of a method for managing therapy plan data provided by another embodiment of the disclosure.
FIG. 3 is a schematic structural diagram of a data exchange device provided by an embodiment of the disclosure.
FIG. 4 is a schematic structural diagram of a system for managing therapy plan data provided by an embodiment of the disclosure.
FIG. 5 is a block diagram of a control system provided by an embodiment of the disclosure.

### DETAILED DESCRIPTION

Clear and intact description will be made on the technical solutions in the embodiments of the disclosure below in combination with drawings required to be used in the embodiments of the disclosure. It is apparent that the drawings described below are merely a part of the embodiments of the disclosure rather than all of the embodiments.

It should be noted that on the basis of the embodiments in the disclosure, all related embodiments obtained by those of ordinary skill in the art without paying any creative work fall into the scope of protection of the disclosure.

The embodiments of the disclosure provide a method and system for managing therapy plan data and a data exchange device, which are described in detail below respectively.

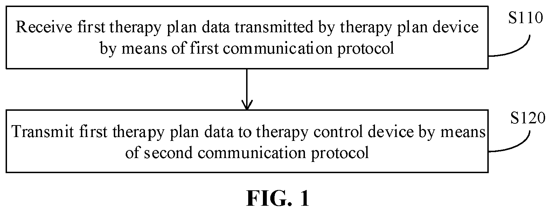

FIG. 1 is a schematic flowchart of a method for managing therapy plan data provided by an embodiment of the disclosure. An executive body of the method may be any device capable of implementing the method, for example, a data exchange device, a data management device and the like. By example of taking the data exchange device as the executive body of the method, the method includes the following operations.

In operation S 110, first therapy plan data transmitted by a therapy plan device by means of a first communication protocol is received.

Specifically, the therapy plan device performs data interaction with the data exchange device by means of the first communication protocol, the therapy plan device transmits the first therapy plan data to the data exchange device by means of the first communication protocol, and the data exchange device receives the first therapy plan data.

It should be understood that the first therapy plan data may include therapy plan data of one target object or multiple target objects. The embodiment of the disclosure does not limit specific content of the first therapy plan data. The first therapy plan data may be either generated in the therapy plan device or stored in the therapy plan device. The embodiment of the disclosure does not limit a source of the first therapy plan data specifically. The therapy plan device may either transmit the first therapy plan data to the data exchange device in real time or transmit the first therapy plan data to the data exchange device at a certain time interval, which is not limited specifically in the embodiment of the disclosure.

In operation S120, the first therapy plan data is transmitted to a therapy control device by means of a second communication protocol.

Specifically, the data exchange device is connected to the therapy control device by means of the second communication protocol, and the data exchange device transmits the first therapy plan data to the therapy control device by means of the second communication protocol. In some embodiments, the data exchange device transmits the first therapy plan data to the therapy control device directly by means of the second communication protocol. In some other embodiments, the data exchange device processes the first therapy plan data, for example, performs data format conversion or compression or the like thereto, and then transmits the first therapy plan data subjected to processing to the therapy control device by means of the second communication protocol. The embodiment of the disclosure does not limit it specifically.

It should be understood that the data exchange device may either transmit the first therapy plan data to the therapy control device in real time or transmit the first therapy plan data to the therapy control device according to an order of the therapy control device. The embodiment of the disclosure does not limit the way by which the data exchange device transmits the first therapy plan data to the therapy control device specifically. The data exchange device may further store the first therapy plan data or process the first therapy plan data, which is not limited specifically in the embodiment of the disclosure.

According to the technical solution provided by the embodiment of the disclosure, first therapy plan data transmitted by a therapy plan device by means of a first communication protocol is received; and the first therapy plan data is transmitted to a therapy control device by means of a second communication protocol, so that data interaction may be performed timely between the therapy plan device and the therapy control device, and problems such as low efficiency, poor maintainability, low system redundancy and the like occurred when data interaction is performed directly between the therapy plan device and the therapy control device are avoided.

In an embodiment of the disclosure, the first communication protocol and the second communication protocol are the same or different, the first communication protocol or the second communication protocol is a communication protocol based on IP; the communication protocol based on IP includes TCP/IP.

It should be understood that the first communication protocol and the second communication protocol may be either the same or different, which is not limited specifically in the embodiment of the disclosure. The first communication protocol or the second communication protocol may be either the communication protocol based on IP or another communication protocol, which is not limited specifically in the embodiment of the disclosure.

In an embodiment of the disclosure, the first communication protocol includes any one of HTTP, HTTPS, FTP or SSH Protocol, and the second communication protocol includes any one of the HTTP, the HTTPS, the FTP or the SSH protocol.

It should be understood that the first communication protocol includes, but is not limited to, any one of the HTTP, the HTTPS, the FTP or the SSH protocol, which is not limited specifically in the embodiment of the disclosure. The second communication protocol includes, but is not limited to, any one of the HTTP, the HTTPS, the FTP or the SSH protocol, which is not limited specifically in the embodiment of the disclosure.

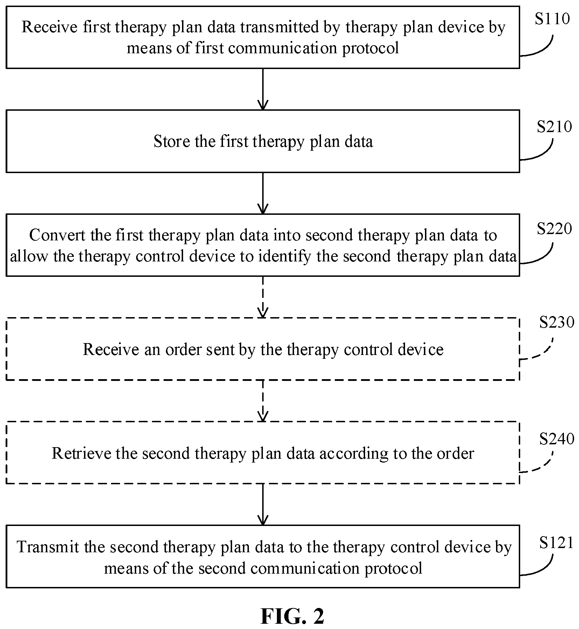

FIG. 2 is a schematic flowchart of a method for managing therapy plan data provided by another embodiment of the disclosure. The embodiment shown in FIG. 2 is a variant of the embodiment shown in FIG. 1. As shown in FIG. 2, its difference from the embodiment shown in FIG. 1 lies in that besides operations S110 and S121 corresponding to the operation S120, the method for managing therapy plan data further includes operations S210 and S220.

In operation S110, first therapy plan data transmitted by a therapy plan device by means of a first communication protocol is received.

In operation S210, the first therapy plan data is stored.

In operation S220, the first therapy plan data is converted into second therapy plan data to allow the therapy control device to identify the second therapy plan data.

Specifically, the data exchange device has a function of data conversion and is capable of converting the first therapy plan data into the second therapy plan data recognizable by the therapy control device.

In some embodiments, the data exchange device may store the first therapy plan data and then converts the first therapy plan data into the second therapy palm data; in some other embodiments, the data exchange device may convert the first therapy plan data into the second therapy plan data and then store the second therapy plan data. A sequence of the operations S210 and S220 is not limited specifically in the embodiment of the disclosure.

It should be understood that the data exchange device may automatically acquire a data format recognizable by the therapy plan device and the therapy control device after it is connected with the therapy plan device and the therapy control device, or in the data exchange device, an operator may convert the data format of the first therapy plan data into a data format of the second therapy plan data recognizable by the therapy control device, according to the data format recognizable by the therapy plan device and the therapy control device, which is not limited specifically in the embodiment of the disclosure. The operation that the first therapy plan data is converted into the second therapy plan data may either be an operation that the data exchange device automatically converts the first therapy plan data into the second therapy plan data after receiving the first therapy plan data, or be an operation that the data exchange device converts the first therapy plan data into the second therapy plan data before it is necessary to transmit the first therapy plan data to the therapy control device, which is not specifically limited in the embodiment of the disclosure. The data exchange device may merely store the first therapy plan data, may further merely store the second therapy plan data, and may further store the first therapy plan data and the second therapy plan data simultaneously, which is not limited specifically in the embodiment of the disclosure.

In operation S121, the second therapy plan data is transmitted to the therapy control device by means of the second communication protocol.

According to the technical solution provided by the embodiment of the disclosure, by storing the first therapy plan data, a condition that the therapy control device is unable to acquire data from the therapy plan device timely as the therapy plan device is affected by external factors such as to malicious data tampering, electricity outage, network outage and the like may be avoided, and a condition that the therapy control device is unable to receive the therapy plan data to make therapy delayed as the therapy plan device makes mistakes as a result of the operator may be avoided. In addition, by converting the first therapy plan data into the second therapy plan data to allow the therapy control device to recognize the second therapy plan data, the range of application of the data exchange device may be expanded, so that the data exchange device may be suitable for therapy plan devices and therapy control devices of various data formats.

In an embodiment of the disclosure, the method further includes operations S230 and S240 before the operation S121.

In operation S230, an order sent by the therapy control device is received.

Specifically, the therapy control device sends an order including retrieval of some therapy plan data to the data exchange device, and the data exchange device receives the order.

It should be understood that the order may include retrieval of some therapy plan data such as the first therapy plan data or the second therapy plan data or the like, and may further include an address of the therapy control device, a target object corresponding to some therapy plan data and the like. The embodiment of the disclosure does not specifically limit the contents included in the order.

In operation S240, the second therapy plan data is retrieved according to the order.

Specifically, the data exchange device searches and retrieves the second therapy plan data from the database or a memory pool or the like of the data exchange device according to the order.

It should be understood that besides the second therapy plan data, the data exchange device may further include other therapy plan data, which is not limited specifically in the embodiment of the disclosure. The operations S230 and S240 may be executed before or after the operation S220. When the operations S230 and S240 may be executed before the operation S220, in the operation S240, the first therapy plan data is retrieved according to the order, which is not limited specifically in the embodiment of the disclosure.

In the embodiment of the disclosure, the order sent by the therapy control device is received and the second therapy plan data is retrieved according to the order, so that unnecessary data transmission is reduced and the efficiency of the therapy control device acquiring the therapy plan data is improved.

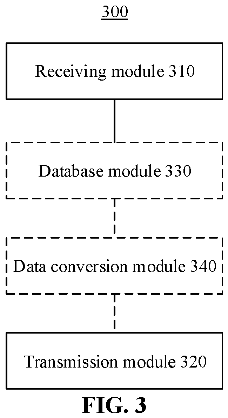

FIG. 3 is a schematic structural diagram of a data exchange device 300 provided by an embodiment of the disclosure. The data exchange device 300 includes a receiving module 310 configured to receive first therapy plan data transmitted by a therapy plan device by means of a first communication protocol, and a transmission module 320 configured to transmitting the first therapy plan data to a therapy control device by means of a second communication protocol.

According to the technical solution provided by the embodiment of the disclosure, by configuring the data exchange device to include the receiving module configured to receive the first therapy plan data transmitted by the therapy plan device by means of the first communication protocol and the transmission module configured to transmit the first therapy plan data to the therapy control device by means of the second communication protocol, the therapy plan data in the therapy plan device may be transmitted to the therapy control device, so that data interaction may be timely performed between the therapy plan device and the therapy control device, and problems such as low efficiency, poor maintainability, low system redundancy and the like occurred when data interaction is performed directly between the therapy plan device and the therapy control device are avoided.

In an embodiment of the disclosure, the data exchange device 300 further includes a database module 330 configured to store the first therapy plan data, herein the receiving module 310 is further configured to send the first therapy plan data to the database module 330, and the transmission module 320 is further configured to retrieve the first therapy plan data from the database module 330.

In an embodiment of the disclosure, the data exchange device 300 further includes a data conversion module 340 configured to convert the first therapy plan data into second therapy plan data to allow the therapy control device to identify the second therapy plan data, herein the transmission module 320 is further configured to transmit the second therapy plan data to the therapy control device by means of the second communication protocol.

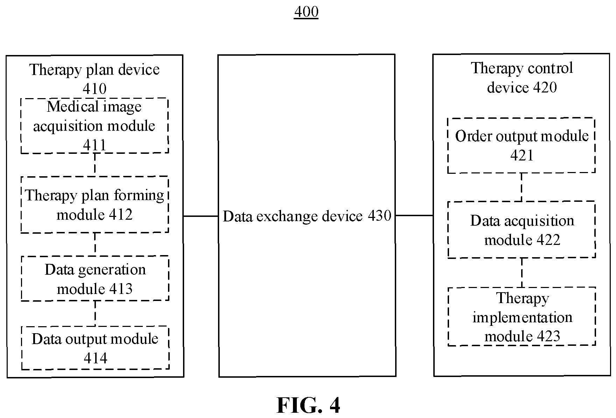

FIG. 4 is a schematic structural diagram of a system 400 for managing therapy plan data provided by an embodiment of the disclosure. As shown in FIG. 4, the system 400 for managing therapy plan data includes: a therapy plan device 410 configured to generate first therapy plan data; a therapy control device 420 configured to control therapy according to the first therapy plan data; and a data exchange device 430 located between the therapy plan device 410 and the therapy control device 420, and configured to receive the first therapy plan data transmitted by the therapy plan device 410 by means of a first communication protocol and transmit the first therapy plan data to the therapy control device 420 by means of a second communication protocol.

It should be understood that the data exchange device 430 may be either any one data exchange device in the embodiment shown in FIG. 3 or a data exchange device obtained by equivalently replacing or substantially deforming any one data exchange device described in the embodiment shown in FIG. 3. The embodiment of the disclosure does not specifically limit the structure of the data exchange device.

According to the technical solution provided by the embodiment of the disclosure, by arranging the data exchange device between the therapy plan device and the therapy control device, as the data exchange device is a device that is not operated manually, it may be ensured that the data exchange device is online for a long time, so that it mey be ensured that the therapy control device may acquire required therapy plan data anytime, the redundancy of the therapy control device acquiring the therapy plan data is improved and delay of acquiring the required therapy plan data due to intermittent operation of the therapy plan device is avoided, or even avoiding the therapy progress being affected. In addition, due to the presence of the data exchange device, the therapy plan device and the therapy control device may operate independently. When the therapy plan device generates the therapy plan data, it is unnecessary to consider problems occurred when the therapy control device manages the therapy plan data; when the therapy control device is developed, it is further unnecessary to consider problems occurred when the therapy plan data is received from the therapy plan device; and when the therapy plan device and the therapy control device are updated, it is further unnecessary to consider the problem that whether one of the devices is compatible with the other one or not, so that the efficiency, the redundancy and the maintainability of the system for managing therapy plan data are improved.

In an embodiment of the disclosure, a therapy plan device 410 includes a medical image acquisition module 411 configured to acquire a medical image, a therapy plan forming module 412 configured to form a therapy plan according to the medical image, a data generation module 413 configured to generate the first therapy plan data according to the therapy plan, and a data output module 414 configured to transmit the first therapy plan data to a receiving module in the data exchange device 430 by means of the first communication protocol. The therapy control device 420 includes an order output module 421 configured to send an order to a transmission module in the data exchange device 430 by means of the second communication protocol, a data acquisition module 422 configured to acquire, by means of the second communication protocol, the first therapy plan data transmitted by the transmission module in the data exchange device 430 according to the order, and a therapy implementation module 423 configured to control therapy according to the first therapy plan data.

In the embodiment of the disclosure, by configuring the therapy plan device to include the medical image acquisition module, the therapy plan forming module, the data generation module and the data output module, the therapy plan device forms a therapy plan according to the medical image and generates the first therapy plan data, and further transmits the first therapy plan data to the data exchange device. In addition, by configuring the therapy control device to include the order output module, the data acquisition module and the therapy implementation module, the first therapy plan data is acquired from the data exchange device to implement therapy.

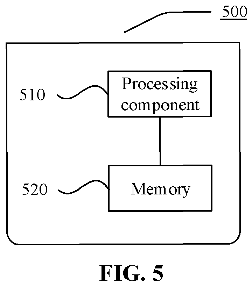

FIG. 5 is a block diagram of a control system 500 provided by an embodiment of the disclosure.

Referring to FIG. 5, the control system 500 includes a processing component 510 which further includes one or more processors, and a memory resource represented by a memory 520 and configured to store instructions, such as an application program, executable by the processing component 510. The application program stored in the memory 520 may include one or more than one modules, each of the modules corresponding to a set of instructions. In addition, the processing component 510 is configured to execute the instructions so as to execute the method for managing therapy plan data.

The control system 500 may further include a power supply component configured to execute power supply management of the control system 500, a wired or wireless network interface configured to connect the control system 500 to a network and an input/output (I/O) interface. The control system 500 may operate based on operating systems stored in the memory 520, for example, Windows Server^{™}, Mac OS X^{™}, Unix^{™}, Linux^{™}, FreeBSD^{™} or the like.

Anon-temporary computer-readable storage medium, allows the control system 500 to execute a method for managing therapy plan data when the instructions in the storage medium are executed by the processor of the control system 500, the method including the following operations, First therapy plan data transmitted by a therapy plan device by means of a first communication protocol is received; and the first therapy plan data is transmitted to a therapy control device by means of a second communication protocol.

Those of ordinary skill in the art may realize that the units and algorithm steps of each example described in combination with the embodiments disclosed in the disclosure may be implemented by electronic hardware or a combination of computer software and the electronic hardware. Execution of these functions by way of hardware or software is dependent on a specific application and a design constraint condition of the technical solution. Professionals may implement the described functions for each specific application by using different methods, and the implementation shall not be considered to go beyond the scope of the disclosure.

When the function is implemented in form of a software function unit and sold or used as an independent product, the function may also be stored in a computer-readable storage medium. Based on such an understanding, the technical solutions of the disclosure substantially or parts making contributions to the conventional art or part of the technical solutions may be embodied in form of software product, and the computer software product is stored in a storage medium, including instructions configured to enable a piece of computer equipment (which may be a personal computer, a server, network equipment or the like) to execute all or part of the operations of the method in each embodiment of the disclosure. The abovementioned storage medium includes: various media capable of storing program codes such as a U disk, a mobile hard disk, a Read-Only Memory (ROM), a Random Access Memory (RAM), a magnetic disk or an optical disk.

Those skilled in the art may clearly learn about that specific working processes of the data exchange device and the control system as described above may refer to the logic process in the embodiments of the method for managing therapy plan data and will not be elaborated herein, for convenient and brief description.

It should be further noted that a combination mode of technical features in the disclosure is not limited to a combination mode recorded in claims of the disclosure or a combination mode recorded in the specific embodiments. All technical features recorded in the disclosure may be combined or united freely in any modes, unless otherwise mutually contradicted.

The above is merely the preferred embodiment of the disclosure and is not intended to limit the disclosure. Any modification, equivalent replacement and the like made within the spirit and principle of the disclosure shall be considered as falling within the protection scope of the disclosure.

## Claims

1. A method for managing therapy plan data, comprising:
receiving first therapy plan data transmitted by a therapy plan device by means of a first communication protocol; and
transmitting the first therapy plan data to a therapy control device by means of a second communication protocol.

2. The method of claim 1, further comprising:
storing the first therapy plan data; and
converting the first therapy plan data into second therapy plan data to allow the therapy control device to identify the second therapy plan data;
wherein the transmitting the first therapy plan data to the therapy control device by means of the second communication protocol comprises:
transmitting the second therapy plan data to the therapy control device by means of the second communication protocol.

3. The method of claim 1 or 2, further comprising:
receiving an order sent by the therapy control device; and
retrieving the first therapy plan data according to the order.

4. The method of claim 1 or 2, wherein the first communication protocol and the second communication protocol are the same or different.

5. The method of claim 1 or 2, wherein the first communication protocol or the second communication protocol is a communication protocol based on Internet Protocol (IP), the communication protocol based on IP comprising a Transmission Control Protocol TCP/IP.

6. The method of claim 1 or 2, wherein the first communication protocol comprises any one of a Hypertext Transfer Protocol (HTTP), a Hypertext Transfer Protocol Secure (HTTPS), a File Transfer Protocol (FTP) or a Secure Shell (SSH) Protocol, and the second communication protocol comprises any one of the HTTP, the HTTPS, the FTP or the SSH protocol.

7. A data exchange device, comprising:
a receiving module configured to receive first therapy plan data transmitted by a therapy plan device by means of a first communication protocol; and
a transmission module configured to transmit the first therapy plan data to a therapy control device by means of a second communication protocol.

8. The data exchange device of claim 7, further comprising:
a database module configured to store the first therapy plan data, wherein the receiving module is further configured to send the first therapy plan data to the database module, and the transmission module is further configured to retrieve the first therapy plan data from the database module.

9. The data exchange device of claim 7 or 8, further comprising:
a data conversion module configured to convert the first therapy plan data into second therapy plan data to allow the therapy control device to identify the second therapy plan data, wherein the transmission module is further configured to transmit the second therapy plan data to the therapy control device by means of the second communication protocol.

10. A system for managing therapy plan data, comprising:
a therapy plan device configured to generate first therapy plan data;
a therapy control device configured to control therapy according to the first therapy plan data; and
a data exchange device located between the therapy plan device and the therapy control device, and configured to receive the first therapy plan data transmitted by the therapy plan device by means of a first communication protocol and transmit the first therapy plan data to the therapy control device by means of a second communication protocol.

11. The system of claim 10, wherein the therapy plan device comprises a medical image acquisition module configured to acquire a medical image, a therapy plan forming module configured to form a therapy plan according to the medical image, a data generation module configured to generate the first therapy plan data according to the therapy plan, and a data output module configured to transmit the first therapy plan data to a receiving module in the data exchange device by means of the first communication protocol.

12. The system of claim 10, wherein the therapy control device comprises an order output module configured to send an order to a transmission module in the data exchange device by means of the second communication protocol, a data acquisition module configured to acquire, by means of the second communication protocol, the first therapy plan data transmitted by the transmission module in the data exchange device according to the order, and a therapy implementation module configured to control therapy according to the first therapy plan data.

13. The system of claim 10, wherein the data exchange device comprises a receiving module configured to receive the first therapy plan data transmitted by the therapy plan device by means of the first communication protocol, and a transmission module configured to transmit the first therapy plan data to the therapy control device by means of the second communication protocol.

14. The system of claim 13, wherein the data exchange device further comprises a database module configured to store the first therapy plan data, wherein the receiving module is further configured to send the first therapy plan data to the database module, and the transmission module is further configured to retrieve the first therapy plan data from the database module.

15. The system of claim 13 or 14, wherein the data exchange device further comprises a data conversion module configured to convert the first therapy plan data into second therapy plan data to allow the therapy control device to identify the second therapy plan data, and wherein the transmission module is further configured to transmit the second therapy plan data to the therapy control device by means of the second communication protocol.
